# EUROPÄISCHE PATENTSCHRIFT

(11) **EP 3 479 848 B1**
(45) Veröffentlichungstag und Bekanntmachung des Hinweises auf die Patenterteilung: **05.10.2022**
(21) Anmeldenummer: 17200414.5
(22) Anmeldetag: 07.11.2017
(51) Int. Cl.: A61L 2/08, B65B 55/08

(54) **OBERFLÄCHEN-DEKONTAMINATIONSVORRICHTUNG SOWIE BETRIEBSVERFAHREN**
SURFACE DECONTAMINATION DEVICE AND METHOD OF OPERATION
DISPOSITIF DE DÉCONTAMINATION DE SURFACE AINSI QUE PROCÉDÉ DE FONCTIONNEMENT

(43) Veröffentlichungstag der Anmeldung: 08.05.2019
(73) Patentinhaber: Metall + Plastic GmbH, 78315 Radolfzell (DE)
(72) Erfinder: Kleinmann, Stefan, 78315 Radolfzell (DE)
(74) Vertreter: Patent- und Rechtsanwälte Behrmann Wagner PartG mbB

(56) Entgegenhaltungen:
- EP-A1- 2 854 138
- WO-A1-2008/055375
- US-A1- 2008 193 341
- Metall + Plastic Gmbh: "E-Beam Tunnel", , 17. Juli 2016 (2016-07-17), XP055472115, Gefunden im Internet: URL:https://www.metall-plastic.com/de-de/e -beam-tunnel [gefunden am 2018-05-03]

## Beschreibung

Die Erfindung betrifft eine Oberflächen-Dekontaminationsvorrichtung gemäß Anspruch 1, insbesondere für pharmatechnische Anwendungen, zur Dekontamination von vorsterilisierte Verbrauchsgüter, wie beispielsweise Fertigspritzen, Arzneiverpackungen, etc., beinhaltenden, einen Umfangsbund aufweisenden Umverpackung vor dem Transfer in einen Isolator, in dem die vorsterilisierten Verbrauchsgüter verarbeitet, insbesondere mit einem pharmazeutischen Wirkstoff od.dgl. gefüllt werden. Das Entpacken der Verbrauchsgüter aus den Umverpackungen kann unmittelbar im Isolator, insbesondere einem Manipulatorraum des Isolators, erfolgen oder einer dem Isolator vorgeordneten, bevorzugt als Schleuse ausgebildeten Reinraum-Entpackungskammer. Das Entpacken der Verbrauchsgüter kann entweder manuell, insbesondere über in einen Entpackungs- bzw. Reinraum hineinreichende Handschuhe oder automatisiert mittels einer entsprechenden Entpackungsvorrichtung erfolgen, wobei zum Öffnen der Umverpackungen bevorzugt eine auf dem Umfangsbund der Umverpackungen gesiegelte Folie entfernt und/oder geöffnet wird. Zusätzlich oder alternativ zu dem vorerwähnten Umfangsbund mit darauf gesiegelter Folie kann der Umfangsbund der Umverpackung von einer, insbesondere umlaufenden Ringschulter in der Umfangswand der Umverpackung gebildet sein, ausgehend von der sich die Umfangswand nach oben in Richtung einer Umverpackungsoberseite, insbesondere Deckelfolie, und gegenüberliegend nach unten in Richtung einer Umverpackungsbodenseite fortsetzt. Die nach dem Konzept der Erfindung ausgebildete Oberflächen-Dekontaminationsvorrichtung ist bevorzugt als Transfervorrichtung ausgebildet. Die Oberflächen-Dekontaminationsvorrichtung umfasst Fördermittel zum Fördern der Umverpackungen in einer Förderrichtung entlang eines Förderweges von einer Eingangsseite der Vorrichtung zu einer Ausgangsseite der Vorrichtung, an welche bevorzugt entweder unmittelbar der Isolator oder eine fakultative Entpackungskammer mit nachfolgendem Isolator anschließt. Der Förderweg, auf dem die Umverpackungen in der Oberflächen-Dekontaminationsvorrichtung gefördert werden führt durch einen zwischen Eingangsseite und Ausgangsseite angeordneten Dekontaminationsraum (Bestrahlungsraum), dem eine Strahlungsquelle (Strahlungsemitter), insbesondere eine Elektrodenstrahlquelle, zum Bestrahlen der durch den Dekontaminationsraum förderbaren bzw. geförderten Umverpackungen zugeordnet ist, wobei die Fördermittel eine im Dekontaminationsraum angeordnete Hängetransporteinrichtung zum hängenden Transportieren der Umverpackungen an ihrem Umfangsbund umfassen, wobei bevorzugt die Hängetransporteinrichtung den Umfangsbund, insbesondere auf zwei einander gegenüberliegenden Seiten, während des Transportes klemmend ausgebildet ist. Ferner führt der Förderweg durch mindestens ein zwischen Eingangsseite und Ausgangsseite angeordneten, zum Dekontaminationsraum benachbarten, insbesondere unmittelbar an diesen angrenzenden, einen Einlass sowie einen Auslass aufweisenden Schleusenraum, wobei bei Einlass und Auslass des Schleusenraums mithilfe von Verschlussmitteln öffen- und schließbar sind, insbesondere wechselweise, um somit einen Strahlenaustritt aus dem Dekontaminationsraum in einen Bereich (je nach Anordnung des Schleusenraums in der Förderrichtung vor oder hinter dem Schleusenraum) zu vermeiden.

Ferner betrifft die Erfindung ein System gemäß Anspruch 8 mit einer erfindungsgemäßen Oberflächen-Dekontaminationsvorrichtung, welcher entlang des Förderweges einem Isolator nachgeordnet ist.

Auch führt die Erfindung auf ein Verfahren zum Betreiben einer erfindungsgemäßen Oberflächen-Dekontaminationsvorrichtung und/oder eines erfindungsgemäßen Systems gemäß des Anspruchs 9. Mit der vermehrten Nutzung vorsterilisierter, insbesondere genesteter Verbrauchsgüter, wie Fertigspritzen für Impfstoffe oder Insulin, wird die Dekontamination der die Verbrauchsgüter beinhaltenden Umverpackungen durch Elektronenbestrahlung zunehmend als Transfer-Technologie zum Überführen der Verbrauchsgüter in einen Isolator eingesetzt, insbesondere da diese Technologie eine kontinuierliche Zuführung in den Isolator erlaubt.

Um die Gefährdung der Benutzer durch die in den Dekontaminationsraum emittierte Strahlung zur Dekontamination der Außenoberfläche der Umverpackungen zu vermeiden, sind unterschiedliche Technologien bekannt geworden. Eine bewährte Technologie sieht vor, die Umverpackungen mittels eines Paternostersystems von einem ersten Höhenniveau auf ein entlang der Vertikalen, um ein Mehrfaches einer Umverpackungsvertikalerstreckung beanstandetes zweites Höhenniveau zu transferieren, auf welchem sich der Dekontaminationsraum mit Strahlungsquelle befindet, wobei die Umverpackungen nach Durchfahren des Dekontaminationsraums wieder auf das erste Höhenniveau verstellt werden. Hierdurch wird ein Labyrinthsystem geschaffen, welches zuverlässig einen Strahlungsaustritt verhindert, da nach zweimaligem Auftreffen bzw. Reflektion von Elektronen an Metalloberflächen ein Großteil der Strahlungsenergie abgebaut wurde. Eine derartige Technologie wurde von der Anmelderin als "E-Beam Tunnel" vorbenutzt.

Die EP 2 854 138 A1 offenbart zudem eine Vorrichtung zur Oberflächen-Dekontamination mit zwei Schleuseneinrichtungen, in der die Handhabung der zu dekontaminierenden Objekte im Bereich einer Strahlungsquelle von zwei in mehreren Dimensionen beweglichen Manipulatoreinrichtungen vorgenommen wird.

Bei einem alternativen System werden die Umverpackungen geradlinig entlang eines horizontalen Förderweges an einer Ringschulter hängend durch den Dekontaminationsraum gefördert, wobei dem Dekontaminationsraum entlang des Förderweges jeweils ein Schleusenraum vor- und nachgeordnet ist, wobei Einlass und Auslass der Schleusenräume wechselseitig mittels jeweils einer vertikal verstellbaren Tür verschließbar sind, sodass zu keinem Zeitpunkt ein freier Strahlungsaustritt möglich ist. Mit dem bekannten System können ohne manuelle Umstellungen nur gleich dimensionierte Umverpackungen verarbeitet werden, da die Umverpackungen bis zum Erreichen des Dekontaminationsraums auf ihrem Boden aufsitzend transportiert werden und dann von der Hängetransporteinrichtung im Dekontaminationsraum übernommen werden - der Höhenabstand der dem Dekontaminationsraum vorgelagerten Bodentransporteinrichtung zu der Hängetransporteinrichtung ist fest vorgegeben und müsste zur Verarbeitung von Umverpackungen mit anderen Dimensionen (falls überhaupt ohne Weiteres möglich) zeitintensiv manuell angepasst werden.

Ein System mit einem gradlinigen, fluchtenden Transportweg ist beispielsweise aus der WO 2008/055375 A1 bekannt, wobei dieses System zudem um horizontale Achsen rotierende Schleusen mit zwei parallelen Platten vorsieht. Die Platten umfassen dabei bezüglich der Rotationsachse gegeneinander versetzte aus Materialbereichen ausgenommene Aussparungen, so dass in eine ersten Winkelposition ein Eintritt in die Schleuse und in einer zweiten Winkelposition ein Austritt aus einer Schleuse möglich ist.

Ähnliche Schleusen, die jedoch um eine vertikale Achse rotieren und mit radial zur Achse verlaufenden senkrechten Abschirmelementen versehen sind, sind zudem aus der US 2008/0193341 A1 bekannt.

Vorstehende Technologien haben sich bewährt. Nachteilig bei der letztgenannten Technologie ist jedoch, dass ein Transport der Umverpackungen, dem die Umverpackungen mit ihrem Verpackungsboden auf einer Fördereinrichtung aufsitzen durch den Dekontaminationsraum hindurch problematisch ist, zum einen, da durch den großflächigen Kontakt der Umverpackungen zu der Fördereinrichtung eine ausreichende Bestrahlung nur schwer realisierbar ist und darüber hinaus die Umverpackungen während des Transports durch den Dekontaminationsraum häufig verkanten. Dies ist auf die vergleichsweise großen Strömungsgeschwindigkeiten im Dekontaminationsraum zurückzuführen, für den wiederum in der Regel isolatorseitig vorgesehener Überdruck bzw. dessen Aufrechterhaltung verantwortlich ist.

Ausgehend von dem vorgenannten Stand der Technik liegt der Erfindung die Aufgabe zugrunde, eine verbesserte, insbesondere als Transfervorrichtung zur Zuführung von Umverpackungen in einen Isolator ausgebildete Oberflächen-Dekontaminationsvorrichtung für die Dekontamination von vorsterilisierte Verbrauchsgüter beinhaltenden Umverpackungen anzugeben, die zur Dekontamination von unterschiedlich dimensionierten, insbesondere eine unterschiedliche Vertikal- bzw. Hocherstreckung aufweisenden Umverpackungen geeignet ist und bei der ohne die Notwendigkeit einer Labyrinthanordnung vorsehen zu müssen ein Strahlungsaustritt in die Umgebung sicher verhindert und gleichzeitig ein sicherer Transport der Umverpackungen durch den Dekontaminationsraum ohne Verkanten sichergestellt ist. Ferner besteht die Aufgabe darin, ein entsprechend verbessertes System sowie ein Betriebsverfahren anzugeben.

Diese Aufgabe wird hinsichtlich der Oberflächen-Dekontaminationsvorrichtung mit den Merkmalen des Anspruchs 1 gelöst, d.h. bei einer gattungsgemäßen Vorrichtung dadurch, dass in den zwei Schleusenräumen je eine Hubeinrichtung zum, insbesondere translatorischen, automatischen vertikalen Verstellen der Umverpackungen relativ zu der Hängetransporteinrichtung angeordnet ist. Bevorzugt ist die Hängetransporteinrichtung dabei derart ausgebildet, dass die Umverpackungen mit ihrem jeweiligen Umverpackungsboden von einem Dekontaminationsraumboden und/oder Dekontaminationsraumeinbauten beabstandet sind, der Boden also frei im Dekontaminationsraum entlang der Strahlungsquelle schwebt.

Hinsichtlich des Systems wird die Aufgabe mit den Merkmalen des Anspruchs 8 gelöst, also derart, dass der nach dem Konzept der Erfindung ausgebildeten Dekontaminationsvorrichtung ein, bevorzugt pharmatechnische Einwendungen ausgebildeter Isolator zum Verarbeiten der aus den Umverpackungen entpackten Verbrauchsgüter im Reinraum, insbesondere in einem Manipulatorraum des Isolators ausgebildet ist. Gegebenenfalls kann zwischen Isolator und Dekontaminationsvorrichtung noch ein Auspackraum zum manuellen oder über eine entsprechende Vorrichtung automatisierten Entpacken der Verbrauchsgüter aus den Umverpackungen angeordnet sein. Alternativ ist es im Rahmen der Erfindung möglich den Isolator derart auszubilden, dass in diesem manuell oder automatisiert über eine entsprechende Vorrichtung die Verbrauchsgüter aus dem dekontaminierten Umverpackungen entpackbar sind. Der Isolator umfasst neben dem Reinraum, insbesondere dem Manipulatorraum zum Verarbeiten, insbesondere Befüllen der Verbrauchsgüter einen, insbesondere des Reinraums angeordneten Umlufterzeugerraum, wobei bevorzugt zwischen Umlufterzeugerraum und Reinraum ein Plenumraum angeordnet ist, der von dem Reinraum über eine Umluft-Laminarisierungsmembran getrennt ist. Bevorzugt weist der Isolator Mittel zum Halten des Reinraums auf einem Überdruck auf.

Hinsichtlich des Verfahrens wird die Aufgabe mit den Merkmalen des Anspruchs 9 gelöst, d.h. bei einem gattungsgemäßen Verfahren dadurch, dass die Umverpackungen an ihrem jeweiligen Umfangsbund hängend mittels einer Hängetransporteinrichtung, insbesondere unter gleichzeitiger Klemmung des Umfangsbundes mittels Klemmmitteln, insbesondere zwischen Rollen und/oder Transportketten, durch den Dekontaminationsraum gefördert werden und in den Schleusenräumen, insbesondere in Abhängigkeit ihrer Vertikalerstreckung, zur Übergabe auf die Hängetransporteinrichtung oder nach der Dekontamination zur Übernahme von der Hängetransporteinrichtung, automatisch vertikal, insbesondere translatorisch, relativ zu der Hängetransporteinrichtung mittels einer im Schleusenraum angeordneten Hubeinrichtung verstellt werden.

Vorteilhafte Weiterbildungen der Erfindung sind in den Unteransprüchen angegeben.

In den Rahmen der Erfindung fallen sämtliche Kombinationen aus zumindest zwei von in der Beschreibung, den Ansprüchen und/oder den Figuren offenbarten Merkmalen.

Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale auch als vorrichtungsgemäß offenbart gelten und beanspruchbar sein.

Der Erfindung liegt der Gedanke zugrunde, entlang des Förderweges vor und nach dem Dekontaminationsraum einen erfindungsgemäßen, eine Hubeinrichtung zum vertikalen Verstellen der Umverpackungen relativ zu einer dekontaminationsraumseitigen Hängetransporteinrichtung aufweisenden Schleusenraum vorzusehen, um mittels der (jeweiligen) Hubeinrichtung einen Höhenunterschied zwischen dem Umverpackungsrand und der Hängetransporteinrichtung automatisch (automatisiert) auszugleichen, um die Umverpackungen für den Fall des Vorsehens eines Schleusenraums entlang des Förderweges in der Förderrichtung vor dem Dekontaminationsraum in einer Übergabeebene auf die Hängetransporteinrichtung durch den Auslass des Schleusenraums hindurch übergeben zu können und im Falle der Anordnung eines solchen Schleusenraums entlang des Förderweges nach der Dekontaminationsanordnung eine Umverpackung in einer Übernahmeebene von der Hängetransporteinrichtung durch den Einlass des Schleusenraums übernehmen und dann nachgeordneten Fördermitteln übergeben zu können. Die Integration einer Hubeinrichtung in einen solchen Schleusenraum ermöglicht eine unproblematische Verarbeitung von unterschiedlich dimensionierten, insbesondere eine unterschiedliche Vertikalerstreckung (Abstanddeckelfolie bis zum Umverpackungsboden) aufweisenden Umverpackungen, da eine entsprechende Höhendifferenz bzw. der jeweils unterschiedliche Abstand zwischen dem Umfangsbund unterschiedlich hoher Umverpackungen zu der Hängetransporteinrichtung noch innerhalb des Schleusenraums überwunden werden kann. Grundsätzlich umfasst die Erfindung eine Oberflächen-Dekontaminationsvorrichtung, bei der sowohl vor als auch nach dem Dekontaminationsraum ein solcher Schleusenraum mit Hubeinrichtung vorgesehen ist. Bevorzugt sind diese Schleusenräume derart ausgebildet, dass diese entweder nacheinander immer nur eine einzige Umverpackung aufnehmen und höhenverstellen können, oder eine Gruppe aus mehreren, entlang des Förderweges hintereinander angeordnete Umverpackungen.

Durch den hängenden Transport der Umverpackungen durch den Dekontaminationsraum kann der Umverpackungsboden frei bleiben und ist somit gut für eine Bestrahlung zugängig. Darüber hinaus werden Verkantungsprobleme, wie sie bei einem Transport auf dem Umverpackungsboden auftreten können sicher vermieden, insbesondere auch da die Flächenpressung im Bereich des flächenkleineren Umfangsbundes deutlich größer ist als am flächengrößeren Umverpackungsboden. Darüber hinaus kann die Hängetransporteinrichtung, wie erwähnt, was bevorzugt ist, mit Klemmmitteln versehen sein, um den Umfangsbund auf zumindest einer Seite während des Transportes zu klemmen, insbesondere zwischen oberen und unteren Transportrollen und/oder zwischen oberen und unteren Transportketten und/oder zwischen mindestens einer Transportrolle und mindestens einer Transportkette. Unter einem hängenden Transport wird jedenfalls verstanden, dass die Gewichtskraft der Umverpackungen, zumindest zum Teil, bevorzugt größtenteils, ganz besonders bevorzugt vollständig während des Transportes durch den Dekontaminationsraum am Umverpackungsumfangsbund abgestützt ist, an dem gemäß einer besonders bevorzugten Ausführungsform eine die Umverpackung verschließende Deckelfolie gesiegelt ist. Auf eine solche Ausführungsform der Umverpackung ist die Erfindung jedoch nicht beschränkt - der Umfangsbund kann auch innerhalb der Umfangswand von einer, insbesondere umlaufenden, Ringschulter gebildet sein, ausgehend von der die sich die Umfangswand sowohl nach oben Richtung Umverpackungsoberseite als auch nach unten in Richtung Umverpackungsboden fortsetzt. Bevorzugt ist dann diese Ringschulter zusätzlich zu einem darüber mit Abstand hierzu angeordneten oberen Umfangsbund vorgesehen, auf dem eine Deckelfolie gesiegelt ist. Der hängende Transport kann alternativ an dem (oberen) Umfangsbund mit Deckelfolie oder einer innerhalb der Umverpackungsumfangswand angeordneten Ringschulter (Umfangsbund) erfolgen.

Die Schleusenräume zeichnen sich durch einen verschließbaren Einlass sowie durch einen verschließbaren Auslass aus, d.h. der Förderweg in den Schleusenraum hinein und aus dem Schleusenraum heraus ist mithilfe von Verschlussmitteln, insbesondere strahlungssicher bzw. -dicht, verschließbar, im einfachsten Fall durch das Vorsehen von zwei, insbesondere vertikal, alternativ seitlich verstellbaren Türen, die bevorzugt derart angesteuert sind, dass Einlass und Auslass im Betrieb nur wechselweise, d.h. nicht gleichzeitig geöffnet sind, um einen Strahlendurchtritt sicher zu vermeiden. Für den Fall des Vorsehens eines Schleusenraums entlang des Förderweges vor dem Dekontaminationsraum dient der Einlass des Schleusenraums zur Hindurchführung von Umverpackungen bzw. zur Übernahme von Umverpackungen von einer vorgelagerten Transporteinrichtung, die die Umverpackungen bevorzugt auf ihrem Boden aufsitzend transportiert. Durch den Auslass eines solchen Schleusenraums gelangen die Umverpackungen nach Vertikalverstellung in den benachbarten Dekontaminationsraum. Für den Fall des Vorsehens eines Schleusenraums entlang des Förderweges nach dem Dekontaminationsraum gelangen die sterilisierten Umverpackungen aus dem Dekontaminationsraum durch den Einlass des Schleusenraums in den Schleusenraum hinein und werden durch den Auslass, insbesondere nach vertikaler Verstellung, bevorzugt nach oben, weiter in Richtung eines Isolators gefördert, bevorzugt wiederum auf dem Umverpackungsboden aufsitzend. Ganz besonders bevorzugt findet also ein Transportsystemwechsel statt, derart, dass bevorzugt die Umverpackungen auf ihrem Umverpackungsboden aufsitzend in einen den Dekontaminationsraum vorgeordneten Schleusenraum hinein gefördert werden, und dann hängend innerhalb des Schleusenraums. Zusätzlich oder alternativ ist es bevorzugt, wenn dekontaminierte Umverpackungen in einem bevorzugt vorgesehenen, den Dekontaminationsraum nachgeordneten Schleusenraum wieder auf ihrem Boden aufsitzen und auf den Boden aufsitzend weiter in Richtung Isolator gefördert werden bzw. förderbar sind.

Insgesamt zeichnet sich die erfindungsgemäße Oberflächen-Dekontaminationsvorrichtung dadurch aus, dass die Umverpackungen bis auf den geringen Höhenversatz in den Schleusenräumen im Wesentlichen geradlinig entlang des Transportweges transportierbar sind, also derart, dass kein einen großen Raumbedarf beanspruchendes Labyrinth gebildet werden muss. Anders ausgedrückt sind die Schleusenräume und der Dekontaminationsraum, d.h. auf zwei voneinander abgewandten Seiten des Dekontaminationsraums angeordnete Schleusenräume, derart angeordnet, dass durch den Einlass und den Auslass beider Schleusenräume sowie durch den Dekontaminationsraum eine gedachte, horizontale Linie verläuft. Darüber hinaus werden Förderprobleme im bevorzugt vorgesehen Luftgegenstrom im Dekontaminationsraum durch den hängenden Transport vermieden, wobei gleichzeitig sichergestellt ist, dass durch das Vorsehen der Hubeinrichtung in den Schleusenräumen eine voneinander unterschiedliche Höhenerstreckung aufweisende Umverpackungen ohne größere Maschinenumstellungen verarbeitbar bzw. in Richtung Isolator transferierbar sind.

Ganz besonders bevorzugt ist eine Ausführungsform der erfindungsgemäßen Vorrichtung und/oder des erfindungsgemäßen Betriebsverfahrens, bei der die Zeit zum Öffnen und/oder Schließen des Schleusenraumeinlasses oder des Schleusenraumauslasses für die Vertikalverstellung der mindestens einen, vorzugsweise ausschließlich einen zu einem jeweiligen Zeitpunkt in dem Schleusenraum befindlichen Umverpackung relativ zu der Hängetransporteinrichtung für eine Übergabe oder Übernahme der Umverpackung auf die bzw. von der Hängetransporteinrichtung genutzt wird. Mit anderen Worten erfolgt die Vertikalverstellung zumindest zum Teil, d.h. beginnt zumindest bevor der Einlass dieses Schleusenraums vollständig geschlossen und/oder der Auslass hin zum Dekontaminationsraum vollständig geöffnet ist. Ganz besonders bevorzugt ist die Hubbewegung vor dem vollständigen Öffnen des Auslasses abgeschlossen. Bei einem dem Dekontaminationsraum nachgeordneten Schleusenraum erfolgt die Verstellung der Umverpackung in vertikaler Richtung bevorzugt zumindest zeitweise, d.h. der Hubvorgang beginnt zumindest, vor einem vollständigen Verschließen des Einlasses (Verbindung zum Dekontaminationsraum) und/oder dem vollständigen Öffnen des Auslasses. Ganz besonders bevorzugt ist die Hubbewegung vor dem vollständigen Öffnen des Auslasses abgeschlossen. Zur vorrichtungsgemäßen Realisierung steuern die Steuermittel die entsprechende Hubeinrichtung und die Verschlussmittel entsprechend an, um eine zeitliche Überschneidung zu erreichen.

Erfindungsgemäß ist vorgesehen, dass die Verschlussmittel eine um eine Rotationsachse verdrehbare, insbesondere metallische, bevorzugt vollumfängliche, Umfangswand mit mindestens einer, bevorzugt ausschließlich einer, in den Förderweg verstellbaren Öffnung umfassen, die derart ausgebildet ist, dass Einlass und Auslass des Schleusenraums ausschließlich wechselweise öffnenbar sind. Mit anderen Worten ist es bevorzugt zur Realisierung eines ausschließlich wechselweise öffenbaren Einlasses und Auslasses eine, bevorzugt bis auf die mindestens eine Durchgangsöffnung zum Hindurchtransport der Umverpackung umfangsgeschlossene Umfangswand rotierbar anzuordnen, die die Hubeinrichtung zumindest abschnittsweise umgibt. Zur Realisierung der wechselweisen Öffenbarkeit von Einlass und Auslass des Schleusenraums ist es bevorzugt, wenn die Umfangswand lediglich eine einzige Durchgangsöffnung aufweist, die je nach Drehstellung entweder den Einlass oder den Auslass bildet. Denkbar ist auch das Vorsehen zweier Durchgangsöffnungen für den Hindurchtransport der Umverpackung, eine erste für zur Bildung des Einlasses und eine zweite zur Bildung des Auslasses - in diesem Fall liegen die Durchgangsöffnungen jedoch bevorzugt nicht diametral einander gegenüber sondern sich bezogen auf diese Diametralposition in Umfangsrichtung versetzt angeordnet, um zu jedem Zeitpunkt sicherzustellen, dass der Förderweg zumindest einlass- oder zumindest auslassseitig durch die Umfangswand blockiert bzw. versperrt ist. Bevorzugt ist die verdrehbare Umfangswand innerhalb einer äußeren Schutzwand, insbesondere aus Metall angeordnet, die die Bewegungsbahn der Umverpackungen in dem sowie aus dem Schleusenraum frei lässt und insbesondere zu zwei Umfangsseiten an den Auslass angrenzt, um bei einem Verschwenken bzw. Verdrehen der Umfangswand sicherzustellen, dass keine Strahlung nach außen gelangen kann.

Im Hinblick auf die konkrete Ausgestaltung der Hubeinrichtung gibt es unterschiedliche Möglichkeiten. Bevorzugt ist die Realisierung eines Linearantriebs, beispielsweise durch das Vorsehen einer pneumatischen oder hydraulischen Kolben-Zylindereinheit, wobei auch alternative Linearantriebe, beispielsweise in Form eines elektromotorischen Spindelantriebs oder eines Zahnriemenantriebs realisierbar sind. Bei der Hubeinrichtung handelt es sich im Wesentlichen bevorzugt um eine Hubjustageeinrichtung, d.h. eine Hubeinrichtung, die verglichen mit der Stand der Technik Labyrinth-Lösung nur einen vergleichsweisen geringen Hub aufweist, da die Umverpackungen im angehobenen Zustand durch den Auslass oder Einlass (je nach Anordnung des Schleusenraums) hin zum bzw. von dem Dekontaminationsraum förderbar sein müssen. Bevorzugt ist der maximale Hub geringer als die Vertikalerstreckung einer maximal mittels der Oberflächen-Dekontaminationsvorrichtung handhabbaren bzw. dekontaminierbaren Umverpackung. Auch ist es möglich, dass die Hubeinrichtung einen vertikal, insbesondere mittels eines Riementriebes, verstellbaren Schlitten umfasst, der bevorzugt an dem Riemen festgelegt ist, wobei der Riemen von einer Antriebswelle angetrieben wird und gegenüberliegend um eine Umlenkwelle geführt ist.

Bevorzugt umfasst die Hubeinrichtung eine Fördereinrichtung der Fördermittel, mit der die Umverpackung (je nach Anordnung des Schleusenraumes vor oder nach dem Dekontaminationsraum) in den Dekontaminationsraum förderbar oder aus diesem heraus förderbar ist. Bevorzugt ist die Fördereinrichtung in eine Hubplattform der Hubeinrichtung integriert und wird somit zusammen mit der Umverpackung in vertikaler Richtung verstellt. Bevorzugt handelt es sich um eine ein Förderband, Förderrollen oder einen Förderriemen umfassende Fördereinrichtung, auf der die Umverpackung mit ihrem Verpackungsboden aufsitzt, sodass zwingend ein Förderprinzipwechsel erfolgt, nämlich von einer anliefernden Förderung, im Rahmen derer sich die Umverpackung mit ihrem Verpackungsboden auf einer Fördereinrichtung abstützt und der hängenden Förderung im Dekontaminationsraum und bevorzugt einer dem Dekontaminationsraum nachgeordneten Förderung wiederum auf dem Umverpackungsboden. Ganz besonders bevorzugt ist eine Ausführungsform, bei der das Förderband, die Förderrollen oder der Förderriemen durch eine vertikale Hohlwelle der Hubeinrichtung hindurch über ein entsprechendes Winkelgetriebe, insbesondere elektromotorisch angetrieben ist bzw. wird.

In Weiterbildung der erfindungsgemäßen Vorrichtung und des erfindungsgemäßen Verfahrens ist es vorgesehen, dass der Verstellweg der mindestens einen Hubeinrichtung und/oder die Strahlungsintensität der mindestens einen Strahlungsquelle und/oder die Fördergeschwindigkeit der Fördermittel, mit welcher die Umverpackungen durch den Dekontaminationsraum förderbar sind bzw. gefördert werden variierbar sind bzw. variiert werden, und zwar in Abhängigkeit eines Umverpackungstyps. Anders ausgedrückt erfolgt die Ansteuerung der Hubeinrichtung und/oder der Strahlungsquelle, insbesondere im Hinblick auf deren Intensität und/oder die Ansteuerung der Fördermittel, insbesondere im Hinblick auf deren Fördergeschwindigkeit in Abhängigkeit unterschiedlicher umverpackungstypspezifischer Ablaufprogramme, wobei jeweils der zu verarbeitende Umverpackungstyp entweder manuell vorgebbar ist, insbesondere durch eine entsprechende umverpackungstypspezifische Programmauswahl und/oder automatisiert, also derart, dass ein jeweiliger Umverpackungstyp sensiert bzw. erkannt wird, beispielsweise durch Sensierung einer Verpackungsgröße oder einer Umverpackungsgröße oder eines umverpackungsgrößenabhängigen Parameters und/oder durch Auslesen eines und/oder in und/oder auf der Verpackung vorgesehenen Codes, wobei dann automatisch in Abhängigkeit des erkannten Umverpackungstyps eines von mehreren Ablaufprogrammen der Steuereinrichtung ausgewählt wird. Denkbar ist es auch, dass Ablaufprogrammparameter unmittelbar in einem Umverpackungscode, beispielsweise einem QR-Code oder Barcode hinterlegt sind.

Für den Fall der Realisierung einer manuellen Ablaufprogrammauswahl umfasst die nach dem Konzept der Erfindung ausgebildete Vorrichtung bevorzugt entsprechende Eingabemittel für einen Benutzer. Für den Fall der automatisierten Ablaufprogrammwahl und/oder der Parametrisierung einer Programmauswahl mit in einem Code hinterlegten Parameter umfasst die Vorrichtung bevorzugt Sensormittel zur automatisierten Detektion unterschiedlicher Verpackungstypen, beispielsweise durch die Erfassung einer Verpackungsgeometrie und/oder durch Auslesen eines Umverpackungscodes.

Weitere Vorteile, Merkmale und Einzelheiten der Erfindung ergeben sich aus der nachfolgenden Beschreibung eines bevorzugten Ausführungsbeispiels sowie anhand der Zeichnung.

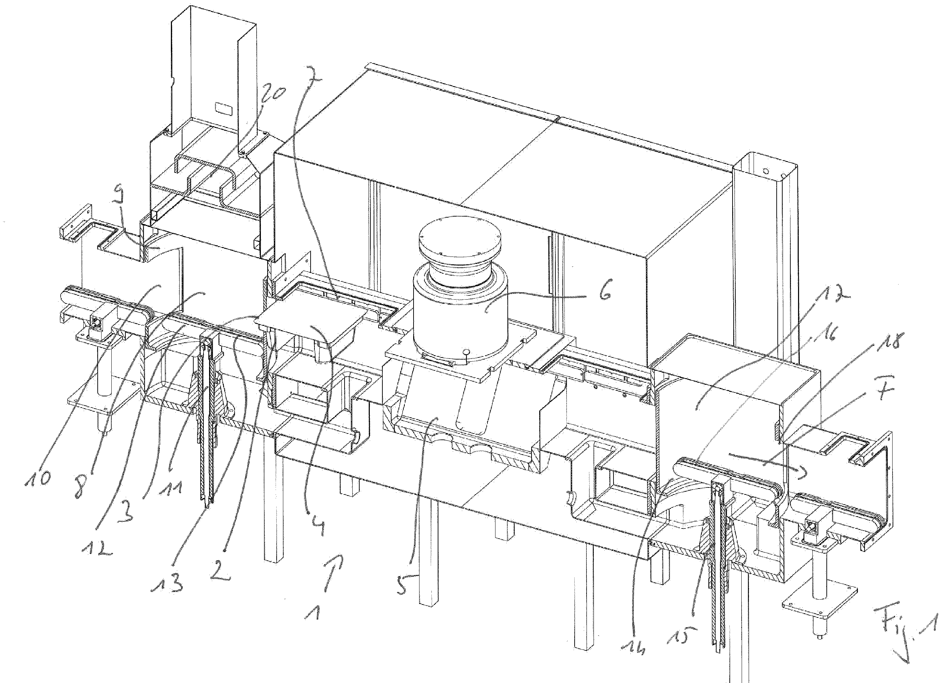

Diese zeigt in der einzigen
- Fig. 1:: eine perspektivische, längsgeschnittene Darstellung eines bevorzugten Ausführungsbeispiels einer nach dem Konzept der Erfindung ausgebildeten Oberflächen-Dekontaminationsvorrichtung zur Durchführung eines erfindungsgemäßen Betriebsverfahrens.

In Fig. 1 ist eine als Transfervorrichtung ausgebildete Oberflächen-Dekontaminationsvorrichtung 1 gezeigt, der im Rahmen eines erfindungsgemäßen Systems ein nicht dargestellter, an sich bekannter Isolator in einer Förderrichtung F nachgeordnet ist. Die Oberflächen-Dekontaminationsvorrichtung 1 dient zum Dekontaminieren von Umverpackungen 2 mit, insbesondere vollumfänglich umlaufendem Umfangsbund 3, auf den eine Deckelfolie 4 gesiegelt ist. Die Umverpackungen 2, von denen in Fig. 1 nur eine einzige exemplarisch dargestellt ist, beinhalten Verbrauchsgüter, wie beispielsweise Fertigspritzen.

Im Zentrum bzw. als Kernbestandteil umfasst die Vorrichtung 1 einen Dekontaminationsraum 5, durch den hindurch die Umverpackungen 2 nacheinander gefördert und dort einer Strahlung, vorliegend einer Elektronenstrahlung ausgesetzt sind. Zu diesem Zweck ist dem Dekontaminationsraum 5 eine (Elektronen-)Strahlenquelle 6 in an sich bekannter Weise zugeordnet. Der Transport der Umverpackungen 2 durch den Dekontaminationsraum 5 erfolgt am Umfangsbund 3 hängend, wobei der Umfangsbund 3 auf zwei einander gegenüberliegenden Seiten von einer nur teilweise dargestellten Hängetransporteinrichtung 7 geklemmt wird, beispielsweise zwischen oberhalb und unterhalb des Umfangsbundes 3 angeordneten Förderrollen und/oder Förderketten.

Bevorzugt ist es, dass innerhalb des Dekontaminationsraums 5 eine überbrückbare bzw. überförderbare Lücke zwischen Einheiten der Hängetransporteinrichtung 7 vorgesehen ist, um auch die ansonsten geklemmten Bereiche für eine Bestrahlung gut zugänglich zu machen.

Dem Dekontaminationsraum 5 ist in der Förderrichtung F ein erster Schleusenraum 8 vorgeordnet, der in dem gezeigten Ausführungsbeispiel eine rotierbare erste Umfangswand 9 aus Metall aufweist, die in dem konkreten Ausführungsbeispiel eine einzige, radiale erste Durchgangsöffnung 10 aufweist, die in der gezeigten Drehposition einen Einlass bildet, um eine Umverpackung 2 zuführen zu können. Auslassseitig ist der erste Schleusenraum 8 geschlossen und damit gegenüber dem Dekontaminationsraum 5 gekapselt. Durch Verdrehen der ersten Umfangswand 9 um 180° wird der Durchgang zum Dekontaminationsraum 5 geöffnet, wobei dann die erste Durchgangsöffnung 10 den Auslass des ersten Schleusenraums 8 bildet, sodass durch den Auslass hindurch eine Umverpackung 2 in den Dekontaminationsraum 5 übergeben werden kann, wobei in dieser Zeit der erste Schleusenraum 8 einlassseitig über die erste Umfangswand 9 verschlossen ist. Innerhalb des ersten Schleusenraums 8 befindet sich eine erste Hubeinrichtung 11 mit einer Hubplattform 12, an der sich eine erste Fördereinrichtung 13, vorliegend in Form eines Bandantriebes befindet, um Umverpackungen 2 von Fördermitteln aus einem Bereich in der Förderrichtung F vor dem ersten Schleusenraum 8 übernehmen und dann nach vertikaler Verstellung nach oben auf die Hängetransporteinrichtung 7 des Dekontaminationsraums 5 übergeben zu können. Die Vertikalverstellung erfolgt dabei bevorzugt zumindest zeitweise bevor der Schleusenraum auslassseitig durch Verdrehen der ersten Umfangswand 9 wieder verschlossen wird.

Dem Dekontaminationsraum 5 ist ein, im Wesentlichen identisch wie der erste Schleusenraum 8 aufgebauter zweiter Schleusenraum 14 mit einer zweiten Hubeinrichtung 15 sowie einer daran angeordneten zweiten Fördereinrichtung 16 nachgeordnet, um Umverpackungen 2 von der Hängetransporteinrichtung 7 übernehmen und dann vertikal nach unten verstellen zu können, um diese dann auf nachgeordnete Fördermittel für den Weitertransport in den Isolator übergeben zu können. Der zweite Schleusenraum 14 umfasst bzw. ist in radialer Richtung begrenzt von eine(r) zweite(n) rotierbare(n) Umfangswand 17 mit einer einzigen zweiten Durchgangsöffnung 18, die wechselseitig bzw. alternativ einen Einlass sprich eine Verbindung zum Dekontaminationsraum 5 bzw. einen Auslass als Verbindung in Richtung Isolator bildet. Bevorzugt erfolgt die Vertikalverstellung der zweiten Hubeinrichtung 15, in diesem Fall nach unten, zumindest zeitweise während des Verdrehens der zweiten Umfangswand 17, d.h. beginnt zumindest vor dem endgültigen Öffnen des Auslasses in Richtung Isolator.

In einem Bereich oberhalb des ersten Schleusenraums 8 befindet sich ein Luftauslass 20 um den aus Richtung des Isolators zuströmenden Luftstrom in einem Bereich in der Förderrichtung F vor dem Dekontaminationsraum 5 abzuführen.

Aus Fig. 1 ergibt sich weiter, dass beide Schleusenräume unabhängig vom Öffnungszustand des Einlassen und des Auslasses dauerhaft luftleitend verbunden sind mit dem Dekontaminationsraum 5, um eine Luftdurchleitung aus dem Isolator entgegen der Förderrichtung F zu gewährleisten.

Den Fördermitteln, insbesondere der Hängetransporteinrichtung 7, sowie den beiden Hubeinrichtungen 11, 12 und der Strahlungsquelle 6 sind Steuermittel (Steuerung, Steuereinrichtung) zugeordnet, die diese Funktionseinheiten in Abhängigkeit eines manuell vorgegebenen oder automatisch erkannten Umverpackungstyps, wie im allgemeinen Beschreibungsteil erläutert, ansteuern, also derart, dass der korrekte Hubweg der Hubeinrichtungen vorgegeben wird, und/oder eine entsprechende Strahlungsintensität der Strahlungsquelle 6 und/oder eine Fördergeschwindigkeit der Hängetransporteinrichtung 7.

### Bezugszeichen

- 1: Vorrichtung
- 2: Umverpackung
- 3: Umfangsbund
- 4: Deckelfolie
- 5: Dekontaminationsraum
- 6: Strahlungsquelle
- 7: Hängetransporteinrichtung
- 8: erster Schleusenraum
- 9: erste Umfangswand
- 10: erste Durchgangsöffnung
- 11: erste Hubeinrichtung
- 12: erste Hubplattform
- 13: erste Fördereinrichtung
- 14: zweiter Schleusenraum
- 15: zweite Hubeinrichtung
- 16: zweite Fördereinrichtung
- 17: zweite Umfangswand
- 18: zweite Durchgangsöffnung

- 20: Luftauslass

- F: Förderrichtung

## Patentansprüche

1. Oberflächen-Dekontaminationsvorrichtung (1) zur Dekontamination von vorsterilisierte Verbrauchsgüter, insbesondere Fertigspritzen, beinhaltenden, einen Umfangsbund (3) aufweisenden Umverpackungen (2) vor dem Transfer in einen Isolator, umfassend Fördermittel zum Fördern der Umverpackungen (2) in einer Förderrichtung (F) entlang eines Förderweges von einer Eingangsseite der Vorrichtung zu einer Ausgangsseite der Vorrichtung (1), wobei der Förderweg durch einen zwischen Eingangsseite und Ausgangsseite angeordneten Dekontaminationsraum (5), dem eine Strahlungsquelle, (6) insbesondere eine Elektronenstahlquelle, zum Bestrahlen der durch den Dekontaminationsraum (5) förderbaren Umverpackungen (2) zugeordnet ist, sowie durch zwei zwischen Eingangsseite und Ausgangsseite angeordnete, zum Dekontaminationsraum (5) benachbarte, einen Einlass sowie einen Auslass aufweisende Schleusenräume (8, 14) hindurch führt, wobei ein erster der beiden Schleusenräume (8) vor und ein zweiter der beiden Schleusenräume (14) nach dem Dekontaminationsraum (5) angeordnet ist, wobei die Einlässe und Auslässe der Schleusenräume mittels Verschlussmitteln öffnen und schließbar sind, wobei die Fördermittel eine im Dekontaminationsraum (5) angeordnete Hängetransporteinrichtung (7) zum hängenden Transportieren der Umverpackungen (2) an ihrem Umfangsbund (3) umfassen,
**dadurch gekennzeichnet,**
**dass** die Verschlussmittel eine um eine Rotationsachse verdrehbare Umfangswand (9, 17) mit mindestens einer, bevorzugt ausschließlich einer, in den Förderweg verstellbaren Öffnung zum Hindurchfördern der Umverpackungen (2) umfassen, die derart ausgebildet ist, dass Einlass und Auslass ausschließlich wechselweise öffnenbar sind, wobei in den Schleusenräumen (8, 14) eine Hubeinrichtung (11, 15) zum automatischen, vertikalen Verstellen der Umverpackungen (2) relativ zu der Hängetransporteinrichtung (7) angeordnet ist.

2. Oberflächen-Dekontaminationsvorrichtung nach Anspruch 1,
**dadurch gekennzeichnet,**
**dass** die Hubeinrichtung (11, 15) einen Linearantrieb, insbesondere eine Kolben-Zylinder-Einheit und/oder einen Zahnriementrieb zum vertikalen Verstellen einer Hubplattform (12) aufweist.

3. Oberflächen-Dekontaminationsvorrichtung nach Anspruch 2,
**dadurch gekennzeichnet,**
**dass** in die Hubeinrichtung (11, 15) eine Fördereinrichtung (F) der Fördermittel, insbesondere mindestens eine Förderolle, ein Förderband oder ein Förderriemen zum, insbesondere horizontalen Fördern von Umverpackungen (2) in Richtung Auslass des Schleusenraums (8) integriert ist, wobei bevorzugt die Fördereinrichtung durch eine vertikale Hohlwelle hindurch antreibbar ist.

4. Oberflächen-Dekontaminationsvorrichtung nach einem der vorhergehenden Ansprüche,
**gekennzeichnet durch**,
eine Steuereinrichtung, die ausgebildet ist zur Ansteuerung der Hubeinrichtung (11, 15) und/oder der Strahlungsquelle (6) und/oder der Fördermittel in Abhängigkeit unterschiedlicher, manuell oder automatisch auswählbarer, umverpackungstypspezifischer, insbesondere umverpackungsdimensionsspezifischer, Ablaufprogramme.

5. Oberflächen-Dekontaminationsvorrichtung Anspruch 4, **gekennzeichnet,**
**durch** Eingabemittel zur manuellen Ablaufprogrammauswahl und/oder Umverpackungstypvorgabe und/oder Umverpackungsdimensionsvorgabe, insbesondere Umverpackungshöhenvorgabe durch einen Benutzer und/oder durch Sensormittel zur automatisierten Detektion unterschiedlicher Umverpackungstypen, insbesondere unterschiedlicher Umverpackungsdimensionen.

6. Oberflächen-Dekontaminationsvorrichtung nach einem der vorhergehenden Ansprüche,
**dadurch gekennzeichnet,**
**dass** der erste Schleusenraum (8) in der Förderrichtung (F) vor dem Dekontaminationsraum (5) angeordnet ist, derart, dass die Umverpackungen (2) aus dessen Auslass in den Dekontaminationsraum (5) förderbar sind und das der zweite Schleusenraum (14) in der Förderrichtung (F) nach dem Dekontaminationsraum (5) angeordnet ist, derart, dass die Umverpackungen (2) aus dem Dekontaminationsraum (5) in den Einlass des Schleusenraums (14) förderbar sind.

7. Oberflächen-Dekontaminationsvorrichtung nach einem der vorhergehenden Ansprüche,
**dadurch gekennzeichnet,**
**dass** die Schleusenräume (8, 14) derart angeordnet sind, dass eine gedachte horizontale Linie durch den Einlass und den Auslass der zwei Schleusenräume (8,14) sowie durch den Dekontaminationsraum (5) verläuft.

8. System, umfassen eine Oberflächen-Dekontaminationsvorrichtung (1) nach einem der vorhergehenden Ansprüche, sowie einen dieser nachgeordneten, bevorzugt pharmatechnischen, Isolator zum Verarbeiten der aus den Umverpackungen (2) entpackten Verbrauchsgüter im Reinraum, insbesondere in einem Manipulatorraum des Isolators.

9. Verfahren zum Betreiben einer Oberflächen-Dekontaminationsvorrichtung (1) nach einem der Ansprüche 1 bis 7 und/oder eines Systems Anspruch 9, wobei einen Umfangsbund (3) aufweisende, bevorzugt auf dem Umfangsbund (3) mit einer Folie verschlossene, Umverpackungen (2) mit darin angeordneten, vorsterilisierte Verbrauchsgütern, insbesondere Fertigspritzen, vor dem Transfer in einen Isolator in einer Förderrichtung (F) entlang eines Förderweges von einer Eingangsseite der Vorrichtung (1) zu einer Ausgangsseite der Vorrichtung (1) gefördert werden, und auf ihrem Förderweg in dem Dekontaminationsraum (5) mit Strahlung, insbesondere Elektronenstrahlung, beaufschlagt werden, wobei die Umverpackungen (2) vor und nach dem Dekontaminationsraum (5) durch einen Einlass und einen Auslass aufweisenden Schleusenraum (8, 14) gefördert werden, dessen Einlass und Auslass zum Verhindern eines Strahlenaustritts mittels Verschlussmitteln geöffnet und geschlossen werden, wobei die Umverpackungen (2) an ihrem jeweiligen Umfangsbund (3) hängend mittels einer Hängetransporteinrichtung (7), insbesondere unter Klemmung des Umfangsbundes (3), durch den Dekontaminationsraum (5) gefördert werden,
**dadurch gekennzeichnet,**
**dass** die Umverpackung (4) im jeweiligen Schleusenraum (8, 14) vor und nach dem Dekontaminationsraum (5), insbesondere in Abhängigkeit ihrer Vertikalerstreckung, zur Übergabe auf die Hängetransporteinrichtung (7) oder nach der Dekontamination (5) zur Übernahme von der Hängetransporteinrichtung (7) automatisch vertikal relativ zu der Hängetransporteinrichtung (7) mittels einer im in den Schleusenräumen (8, 14) angeordneten Hubeinrichtung (11, 15) verstellt werden, wobei die Verschlussmittel eine um eine Rotationsachse verdrehbare Umfangswand (9, 17) mit mindestens einer, bevorzugt ausschließlich einer, in den Förderweg verstellbaren Öffnung zum Hindurchfördern der Umverpackungen (2) umfassen, die derart ausgebildet ist, dass Einlass und Auslass ausschließlich wechselweise öffnen.

10. Verfahren nach Anspruch 9,
**dadurch gekennzeichnet,**
**dass** die Umverpackungen (2) innerhalb der Schleusenräume (8, 14) zumindest zeitweise, bevorzugt vollständig, während des Öffnen und/oder Schließens des Einlasses und/oder des Auslasses mittels der Hubeinrichtung (11, 15) vertikal verstellt werden.

## Claims

1. A surface decontamination device (1) for decontaminating outer packaging (2) which contains pre-sterilized consumer goods, in particular prefilled syringes, and which has a circumferential collar (3) prior to transfer into an isolator, said surface decontamination device (1) comprising conveying means for conveying the outer packaging (2) in a conveying direction (F) along a conveying path from an inlet side of the device to an outlet side of the device (1), the conveying path passing through a decontamination chamber (5) which is disposed between the inlet side and the outlet side and to which a radiation source (6), in particular an electron beam source, is assigned for irradiating the outer packaging (2) which can be conveyed through the decontamination chamber (5), and passing through two lock chambers (8, 14) which are disposed between the inlet side and the outlet side and which are adjacent to the decontamination chamber (5) and which have an inlet and an outlet, a first of the two lock chambers (8) being disposed upstream and a second of the two lock chambers (14) being disposed downstream of the decontamination chamber (5), the inlets and outlets of the lock chambers being able/configured to be opened and closed by means of closing means, the conveying means comprising a suspension transport device (7) disposed in the decontamination chamber (5) for the suspended transport of the outer packaging (2) by its circumferential collar (3),
**characterized in that**
the closing means comprise a circumferential wall (9, 17) which can be rotated about an axis of rotation and which has at least one, preferably solely one, opening which can be displaced into the conveying path for conveying the outer packaging (2) through said circumferential wall (9, 17), the opening being realized in such a manner that the inlet and the outlet can be opened solely in an alternating manner, a lifting device (11, 15) for the automatic, vertical displacement of the outer packaging (2) relative to the suspension transport device (7) being disposed in the lock chambers (8, 14).

2. The surface decontamination device according to claim 1,
**characterized in that**
the lifting device (11, 15) has a linear drive, in particular a piston-cylinder unit and/or a toothed belt drive, for the vertical displacement of a lifting platform (12).

3. The surface decontamination device according to claim 2,
**characterized in that**
a conveying device (F) of the conveying means, in particular at least one conveyor roller, a conveyor belt or a conveyor strap, is integrated into the lifting device (11, 15) for conveying outer packaging (2), in particular in a horizontal manner, towards the outlet of the lock chamber (8), the conveying device being preferably drivable through a vertical hollow shaft.

4. The surface decontamination device according to any one of the preceding claims,
**characterized by**
a control device which is realized for controlling the lifting device (11, 15) and/or the radiation source (6) and/or the conveying means depending on different sequential programs for specific types of outer packaging, in particular specific dimensions of outer packaging, which can be manually or automatically selected.

5. The surface decontamination device according to claim 4,
**characterized by**
input means for the manual selection of the sequential program and/or the specification of the type of outer packaging and/or the specification of the dimensions of the outer packaging, in particular the specification of the height of the outer packaging, by a user and/or **characterized by** sensor means for the automated detection of different types of outer packaging, in particular different dimensions of outer packaging.

6. The surface decontamination device according to any one of the preceding claims,
**characterized in that**
the first lock chamber (8) is disposed upstream of the decontamination chamber (5) in the conveying direction (F), in such a manner that the outer packaging (2) can be conveyed from the outlet of the lock chamber (8) into the decontamination chamber (5) and **in that** the second lock chamber (14) is disposed downstream of the decontamination chamber (5) in the conveying direction (F), in such a manner that the outer packaging (2) can be conveyed from the decontamination chamber (5) into the inlet of the lock chamber (14).

7. The surface decontamination device according to any one of the preceding claims,
**characterized in that**
the lock chambers (8, 14) are disposed in such a manner that an imaginary horizontal line runs through the inlet and the outlet of the two lock chambers (8, 14) and through the decontamination chamber (5).

8. A system comprising a surface decontamination device (1) according to any one of the preceding claims, and a preferably pharmaceutical isolator which is disposed downstream of said surface decontamination device (1) for processing the consumer goods unpacked from the outer packaging (2) in the clean room, in particular in a manipulator chamber of the isolator.

9. A method for operating a surface decontamination device (1) according to any one of claims 1 to 7 and/or a system according to claim 9, outer packaging (2) having a circumferential collar (3) and being preferably closed on the circumferential collar (3) by means of a film and containing pre-sterilized consumer goods disposed therein, in particular prefilled syringes, being conveyed from an inlet side of the device (1) to an outlet side of the device (1) in a conveying direction (F) along a conveying path prior to transfer into an isolator and being exposed to radiation, in particular electron radiation, on the conveying path in the decontamination chamber (5), the outer packaging (2) being conveyed through a lock chamber (8, 14) having an inlet and an outlet and being disposed upstream and downstream of the decontamination chamber (5), the inlet and the outlet of the lock chamber (8, 14) being opened and closed by means of closing means in order to prevent radiation from escaping, the outer packaging (2) being conveyed through the decontamination chamber (5) while being suspended by its respective circumferential collar (3) by means of a suspension transport device (7), in particular with the circumferential collar (3) being clamped,
**characterized in that**
the outer packaging (4) is automatically displaced in a vertical manner relative to the suspension transport device (7) in the respective lock chamber (8, 14) upstream and downstream of the decontamination chamber (5), in particular depending on its vertical orientation, for the transfer onto the suspension transport device (7) or, after the decontamination (5), for the transfer from the suspension transport device (7) by means of a lifting device (11, 15) disposed in the lock chambers (8, 14), the closing means comprising a circumferential wall (9, 17) which can be rotated about an axis of rotation and which has at least one, preferably solely one, opening which can be displaced into the conveying path for conveying the outer packaging (2) through said circumferential wall (9, 17), the opening being realized in such a manner that the inlet and the outlet open solely in an alternating manner.

10. The method according to claim 10,
**characterized in that**
the outer packaging (2) is at least temporarily, preferably entirely, displaced within the lock chambers (8, 14) in a vertical manner by means of the lifting device (11, 15) when the inlet and/or the outlet is/are being opened and/or closed.

## Revendications

1. Dispositif de décontamination de surfaces (1) destiné à décontaminer des suremballages (2) qui contiennent des biens de consommation préstérilisés, notamment des seringues prêtes à l'emploi, et qui comportent un collier circonférentiel (3) avant un transfert dans un isolateur, ledit dispositif de décontamination de surfaces (1) comprenant des moyens de transport destinés à transporter les suremballages (2) dans une direction de transport (F) le long d'un trajet de transport à partir d'un côté d'entrée du dispositif jusqu'à un côté de sortie du dispositif (1), ledit trajet de transport passant par une chambre de décontamination (5) qui est disposée entre le côté d'entrée et le côté de sortie et auquel une source de radiation (6), notamment une source de faisceaux d'électrons, est associée pour irradier les suremballages (2) qui peuvent être transportés à travers la chambre de décontamination (5) et passant par deux chambres de sas (8, 14) qui sont disposées entre le côté d'entrée et le côté de sortie et qui sont adjacentes à la chambre de décontamination (5) et qui comportent une entrée et une sortie, une première chambre parmi les deux chambres de sas (8) étant disposée devant la chambre de décontamination (5) et une deuxième chambre parmi les deux chambres de sas (8) étant disposée derrière la chambre de décontamination (5), les entrées et sorties des chambre de sas pouvant être ouvertes et fermées à l'aide de moyens de fermeture, lesdits moyens de transport comprenant un dispositif de transport suspendu (7) qui est disposé dans la chambre de décontamination (5) et destiné à transporter les suremballages (2) à leurs colliers circonférentiels de manière suspendue,
**caractérisé en ce que**
les moyens de fermeture comprennent une paroi circonférentielle (9, 17) qui peut être tordue autour d'un axe de rotation et qui a au moins une ouverture, de préférence seulement une ouverture, qui peut être déplacée dans le trajet de transport et qui est destinée à transporter les suremballages (2) à travers la même, et qui est réalisée de telle manière que l'entrée et la sortie peuvent être ouvertes seulement tour à tour, un dispositif de levage (11, 15) pour déplacer les suremballages (2) automatiquement et verticalement par rapport au dispositif de transport suspendu (7) étant disposé dans les chambres de sas (8, 14).

2. Dispositif de décontamination de surfaces (1) selon la revendication 1,
**caractérisé en ce que**
le dispositif de levage (11, 15) comporte un entraînement linéaire, notamment une unité piston-cylindre et/ou un entraînement par courroie crantée, destiné à déplacer une plateforme de levage (12) verticalement.

3. Dispositif de décontamination de surfaces (1) selon la revendication 2,
**caractérisé en ce**
**qu'**un dispositif de transport (F) des moyens de transport, notamment au moins un rouleau de transport, une bande transporteuse ou une courroie transporteuse, est intégré dans le dispositif de levage (11, 15), ledit dispositif de transport étant destiné à transporter des suremballages (2), notamment horizontalement, vers la sortie de la chambre de sas (8) et ledit dispositif de transport de préférence étant configuré pour être entraîné à travers un arbre creux vertical.

4. Dispositif de décontamination de surfaces (1) selon l'une quelconque des revendications précédentes,
**caractérisé par**
un dispositif de commande qui est réalisé pour actionner le dispositif de levage (11, 15) et/ou la source de radiation (6) et/ou les moyens de transport en fonction de programmes séquentiels différents pour des sortes spécifiques de suremballages, notamment pour des dimensions spécifiques de suremballages, lesdits programmes séquentiels pouvant être sélectionnés manuellement ou automatiquement.

5. Dispositif de décontamination de surfaces (1) selon la revendication 4,
**caractérisé par**
des moyens d'entrée pour une sélection manuelle d'un programme séquentiel et/ou une prédéfinition de la sorte des suremballages et/ou une prédéfinition des dimensions des suremballages, notamment une prédéfinition de la hauteur des suremballages, par un utilisateur et/ou **caractérisé par** des moyens de capteur destinés à la détection automatique de sortes différentes de suremballages, notamment de dimensions différentes des suremballages.

6. Dispositif de décontamination de surfaces (1) selon l'une quelconque des revendications précédentes,
**caractérisé en ce que**
la première chambre de sas (8) est disposée en amont par rapport à la chambre de décontamination (5) dans la direction de transport (F), de telle manière que les suremballages (2) peuvent être transportés à partir de la sortie de la chambre de sas (8) dans la chambre de décontamination (5), et **en ce que** la deuxième chambre de sas (14) est disposée en aval par rapport à la chambre de décontamination (5) dans la direction de transport (F), de telle manière que les suremballages (2) peuvent être transportés à partir de la chambre de décontamination (5) dans l'entrée de la chambre de sas (14).

7. Dispositif de décontamination de surfaces (1) selon l'une quelconque des revendications précédentes,
**caractérisé en ce que**
les chambres de sas (8, 14) sont disposées de telle manière qu'une ligne horizontale imaginaire traverse l'entrée et la sortie des deux chambres de sas (8, 14) et la chambre de décontamination (5).

8. Système comprenant un dispositif de décontamination de surfaces (1) selon l'une quelconque des revendications précédentes et un isolateur, de préférence de la technique pharmaceutique, qui est disposé en aval par rapport audit dispositif de décontamination de surfaces (1) et destiné à traiter les biens de consommation déballés des suremballages (2) dans la salle blanche, notamment dans une salle de manipulateur de l'isolateur.

9. Procédé destine à opérer un dispositif de décontamination de surfaces (1) selon l'une quelconque des revendication 1 à 7 et/ou un système selon la revendication 9, des suremballages (2) qui comportent un collier circonférentiel (3) et sont de préférence scellés sur le collier circonférentiel (3) au moyen d'un film et contiennent des biens de consommation préstérilisés disposés dans ceux-ci, notamment des seringues prêtes à l'emploi, étant transportés dans une direction de transport (F) le long d'un trajet de transport à partir d'un côté d'entrée du dispositif (1) jusqu'à un côté de sortie du dispositif (1) avant un transfert dans un isolateur, et étant irradiés dans la chambre de décontamination (5), sur leur trajet de transport, notamment avec des faisceaux d'électrons, les suremballages (2) étant transportés à travers une chambre de sas (8, 14) comportant une entrée et une sortie devant et derrière la chambre de décontamination (5), l'entrée et la sortie de la chambre de sas (8, 14) étant ouvertes et fermées à l'aide de moyens de fermeture afin d'empêcher que de la radiation s'échappe, les suremballages (2) étant transportés à travers la chambre de décontamination (5) à l'aide d'un dispositif de transport suspendu (7), étant suspendus à leurs colliers circonférentiels (3) respectifs, notamment en serrant les colliers circonférentiels (3),
**caractérisé en ce que**
le suremballage (4) est automatiquement déplacé dans la chambre de sas (8, 14) respective devant et derrière la chambre de décontamination (5) verticalement par rapport au dispositif de transport suspendu (7), notamment en fonction de son orientation verticale, pour le transfert sur le dispositif de transport suspendu (7) ou après la décontamination (5) pour être pris du dispositif de transport suspendu (7) à l'aide d'un dispositif de levage (11, 15) disposé dans les chambres de sas (8, 14), les moyens de fermeture comprenant une paroi circonférentielle (9, 17) qui peut être tordue autour d'un axe de rotation et qui a au moins une ouverture, de préférence seulement une ouverture, qui peut être déplacée dans le trajet de transport et qui est destinée à transporter les suremballages (2) à travers la même, et qui est réalisée de telle manière que l'entrée et la sortie s'ouvrent seulement tour à tour.

10. Procédé selon la revendication 9,
**caractérisé en ce que**
les suremballages (2) sont déplacés verticalement au moins temporairement, de préférence complètement, dans les chambres de sas (8, 14) à l'aide du dispositif de levage (11, 15) lorsque l'entrée et/ou la sortie est/sont ouverte(s) et/ou fermée(s).
